# EUROPEAN PATENT SPECIFICATION

(11) **EP 4 397 284 B1**
(45) Date of publication and mention of the grant of the patent: **08.07.2026**
(21) Application number: 21955974.7
(22) Date of filing: 01.09.2021
(51) Int. Cl.: A61F 2/90

(54) **STENT DEVICE AND STENT DELIVERY SYSTEM**
STENTVORRICHTUNG UND STENTEINFÜHRUNGSSYSTEM
DISPOSITIF D'ENDOPROTHÈSE ET SYSTÈME DE POSE D'ENDOPROTHÈSE

(43) Date of publication of application: 10.07.2024
(73) Proprietor: Olympus Corporation, Hachioji-shi Tokyo 192-8507 (JP)
(72) Inventor: NOGUCHI, Shun, Tokyo 151-0072 (JP); AOKI, Yu, Tokyo 151-0072 (JP); WATANABE, Yoshihiko, Tokyo 151-0072 (JP); HASEGAWA, Mamoru, Hachioji-shi, Tokyo 192-8507 (JP); WAKABAYASHI, Toru, Hachioji-shi, Tokyo 192-8507 (JP)
(74) Representative: Gunzelmann, Rainer
(86) International application number: PCT/JP2021/032146
(87) International publication number: WO 2023/032084

(56) References cited:
- WO-A1-2014/010679
- WO-A1-2021/166156
- GB-A- 2 346 559
- JP-A- 2007 505 687
- US-A1- 2005 222 667
- US-A1- 2007 112 411
- US-A1- 2017 143 467

## Description

### [Technical Field]

The present invention relates to a stent device and a stent delivery system.

### [Background Art]

A technique for placing a stent in a stenosis or occlusion (hereinafter referred to as a "stenosis or the like") that occurs in the gastrointestinal tract or the like and expanding it is known. A stent delivery system is used to place a stent in the stenosis or the like. The stent delivery system is inserted into a treatment instrument channel of an endoscope to deliver the stent to the stenosis or the like.

A stent device (a covered stent) in which a stent is covered with a thin film (a cover) is used. The stent device covered with the film (the cover) can prevent cells and blood from infiltrating into the stent at the stenosis or the like.

Patent Document 1 describes a covered stent that easily maintains its bent state as it is. The covered stent described in Patent Document 1 is formed by bonding a tape located on the outside and a tape located on the inside through a diamond-shaped space between wires.

### [Citation List]

### [Patent Document]

### [Patent Document 1]

Published Japanese Translation No. 2017-522091 of the PCT International Publication

### [Patent document 2]

US 2005/222667 A1 relates to a stent-graft assembly provided for a variety of medical treatments. The stent-graft assembly includes a stent disposed between an inner layer of graft material and an outer layer of graft material. The graft layers are effectively secured to the stent by attaching the graft layers together through openings in the structure of the stent. Relative movement between the graft layers and the stent is made possible by providing unattached margins between the attached areas of the graft layers and the edges of the stent openings.

### [Summary of Invention]

### [Technical Problem]

However, in the covered stent described in Patent Document 1 and the like, a bonding portion between the tape located on the outside and the tape located on the inside may impede movement of the wire. If the movement of the wire is impeded by the bonding portion, the covered stent will be difficult to curve.

In view of the above circumstances, an object of the present invention is to provide a stent device that is easily curved even when a wire is covered with a thin film (a cover), and a stent delivery system equipped with the stent device.

### [Solution to Problem]

In order to solve the above problems, the present invention proposes the following means.

According to a first aspect of the present invention, there is provided a stent device including: a stent formed in a cylindrical shape by weaving a wire; and a cover formed by overlapping an inside cover disposed on an inside of the stent and an outside cover disposed on an outside of the stent, wherein the cover has a fixation portion at which the inside cover and the outside cover are fixed to each other, and an accommodation portion which is formed by the inside cover and the outside cover and accommodates the wire, and wherein the accommodation portion has a normal accommodation portion and an expanded accommodation portion that is wider than the normal accommodation portion in a radial direction.

According to a second aspect of the present invention, there is provided a stent delivery system including: an operation part; an outer tube member configured to extend to a distal side from the operation part; an inner tube member configured to extend to a distal side from the operation part and located on an inside of the outer tube member; and the above-described stent device which is accommodated between the outer tube member and the inner tube member, wherein the operation part is configured to place the stent device by moving the outer tube member or the inner tube member in a longitudinal direction.

### [Advantageous Effects of Invention]

The stent device of the present invention is easily curved even when a wire is covered with a thin film (a cover).

### [Brief Description of Drawings]

FIG. 1 is a diagram showing the overall configuration of an endoscope system including a stent device according to a first embodiment of the present invention.
FIG. 2 is a diagram showing the overall configuration of the same stent device.
FIG. 3 is a diagram showing the overall configuration of a stent of the same stent device.
FIG. 4 is a development view of the same stent in an F2 region shown in FIG. 3.
FIG. 5 is a diagram showing the same stent device in an F1 region shown in FIG. 2.
FIG. 6 is a cross-sectional view along line S1-S1 shown in FIG. 5.
FIG. 7 is a cross-sectional view along line S2-S2 shown in FIG. 5.
FIG. 8 is a partially transparent view of the same stent device curved to one side.
FIG. 9 is a cross-sectional view along line S3-S3 shown in FIG. 8.
FIG. 10 is a partially transparent view of the same stent device curved to the other side.
FIG. 11 is a cross-sectional view along line S4-S4 shown in FIG. 10.
FIG. 12 is a diagram showing a modification example of an expanded accommodation portion of the same stent device.
FIG. 13 is a cross-sectional view of a stent device according to a second embodiment of the present invention.
FIG. 14 is a cross-sectional view of the same stent device.
FIG. 15 is a cross-sectional view of the same stent device.
FIG. 16 is a cross-sectional view of a stent device according to a third embodiment of the present invention.
FIG. 17 is a cross-sectional view of the same stent device.
FIG. 18 is a cross-sectional view of the same stent device.
FIG. 19 is a cross-sectional view of a modification example of the same stent device.
FIG. 20 is a cross-sectional view of the same modification example of the same stent device.
FIG. 21 is a cross-sectional view of another modification example of the same stent device.
FIG. 22 is a cross-sectional view of the same modification example of the same stent device.
FIG. 23 is a development view of a stent device according to a fourth embodiment of the present invention.
FIG. 24 is a developed view of a portion of a stent device according to a fifth embodiment of the present invention.
FIG. 25 is a diagram showing a portion of the same stent device twisted.
FIG. 26 is a development view of a modification example of the same stent device.

### [Description of Embodiments]

### (First embodiment)

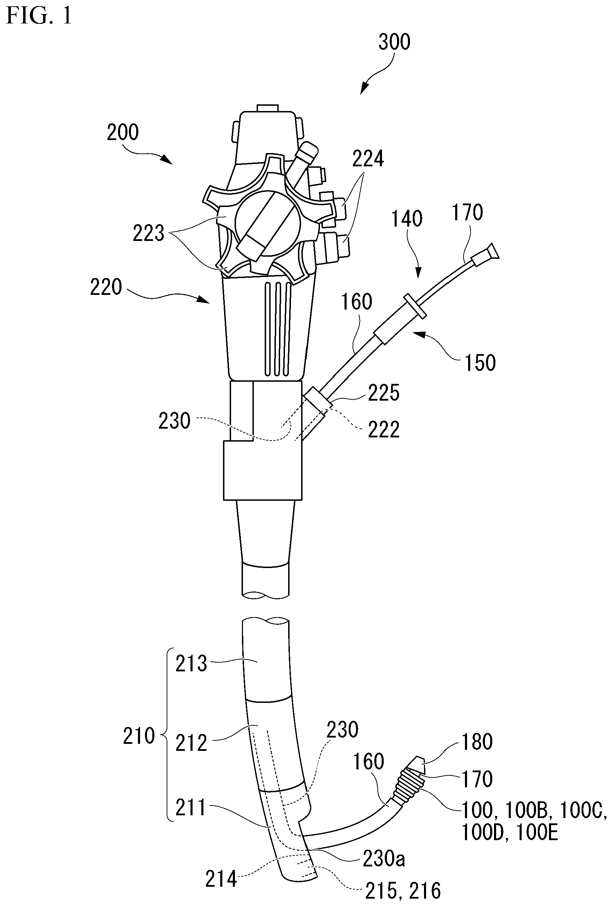

An endoscope system 300 equipped with a stent device 100 according to a first embodiment of the present invention will be described with reference to FIGS. 1 to 11. FIG. 1 is a diagram showing the overall configuration of the endoscope system 300.

### [Endoscope system 300]

The endoscope system 300 includes an endoscope 200 and a stent delivery system 150 inserted into a channel of the endoscope 200.

### [Endoscope 200]

The endoscope 200 is a known side-viewing flexible endoscope and includes a long insertion section 210 and an operation section 220 provided at the proximal end portion of the insertion section 210. The endoscope 200 may be a direct viewing flexible endoscope.

The insertion section 210 includes a distal end rigid portion 211 provided at the distal end portion thereof, a curving portion 212 which is provided on the proximal end side of the distal end rigid portion 211 and which can be curved, and a flexible tube portion 213 provided on the proximal end side of the curving portion 212. A light guide 215 and an imaging unit 216 having a CCD are provided on a side surface of the distal end rigid portion 211 and exposed to the outside.

A treatment instrument channel 230 through which a treatment instrument for an endoscope such as the stent delivery system 150 is inserted is formed in the insertion section 210. A distal end portion 230a of the treatment instrument channel 230 opens on the side surface of the distal end rigid portion 211. A proximal end portion of the treatment instrument channel 230 extends to the operation section 220.

The distal end rigid portion 211 of the treatment instrument channel 230 is provided with a rising base 214. A proximal end portion of the rising base 214 is rotatably supported by the distal end rigid portion 211. A rising base operation wire (not shown) fixed to a distal end portion of the rising base 214 extends to the proximal end side through the insertion section 210.

The curving portion 212 is configured to be able to be freely curved in an up-down direction and a left-right direction. A distal end of the operation wire is fixed to the distal end side of the curving portion 212. The operation wire extends through the insertion section 210 to the operation section 220.

The proximal end side of the operation section 220 is provided with a knob 223 for operating the operation wire and a switch 224 for operating the imaging unit 216 and the like. A user can curve the curving portion 212 in a desired direction by operating the knob 223.

A forceps port 222 that communicates with the treatment instrument channel 230 is provided on the distal end side of the operation section 220. The user can insert a treatment instrument for an endoscope such as the stent delivery system 150 through the forceps port 222. A forceps plug 225 is attached to the forceps port 222 in order to prevent leakage of a bodily fluid.

### [Stent delivery system 150]

The stent delivery system 150 is formed to be elongated as a whole and includes a stent device 100, an outer tube member 160, an inner tube member 170, and an operation part 140.

The outer tube member 160 is formed of a resin or the like, has a cylindrical shape, and has flexibility. The outer tube member 160 can be inserted into the treatment instrument channel 230 of the endoscope 200.

The inner tube member 170 has an outer diameter smaller than the inner diameter of the outer tube member 160 and can pass through the internal space (the lumen) of the outer tube member 160. The inner tube member 170 is formed of a resin or the like and has flexibility. A tip 180 having an outer diameter larger than the outer diameter of the outer tube member 160 is provided at the distal end of the inner tube member 170.

The stent device 100 is accommodated in the distal end portion of the stent delivery system 150, as shown in FIG. 1. The stent device 100 is accommodated in a gap between the inner tube member 170 and the outer tube member 160 in a state in which the inner tube member 170 is passed therethrough and the diameter thereof is reduced.

The operation part 140 is connected to the proximal end sides of the outer tube member 160 and the inner tube member 170, and is configured to be able to move the outer tube member 160 relative to the inner tube member 170 in a longitudinal direction. By operating the operation part, the operator moves the outer tube member 160 relative to the inner tube member 170 to expose the accommodated stent device 100, and as a result, the stent device 100 can be placed. Furthermore, when the stent has been exposed, the operator can re-accommodate the stent device 100 in the gap between the inner tube member 170 and the outer tube member 160 by operating the outer tube member 160 to move relative to the inner tube member 170 in an opposite direction.

### [Stent device 100]

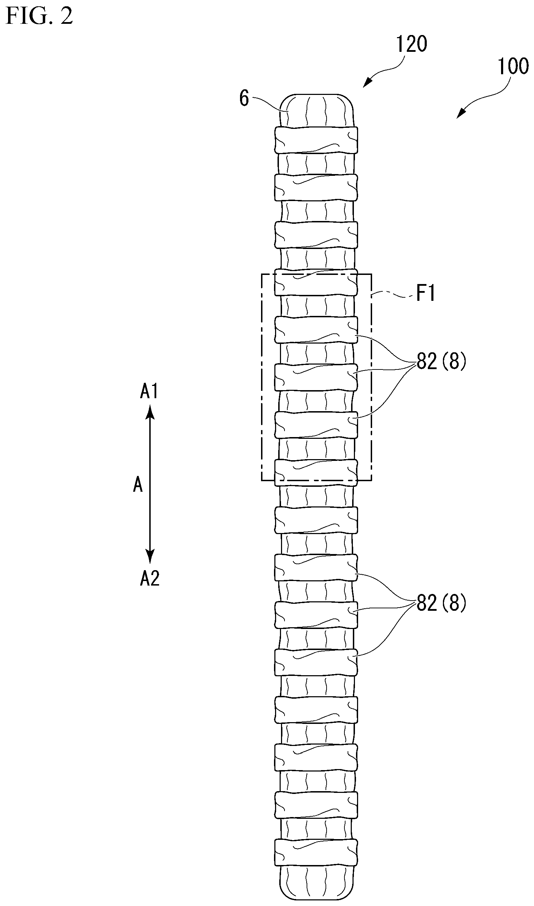

FIG. 2 is a diagram showing the overall configuration of the stent device 100.

The stent device 100 is placed in a body lumen of a digestive system, such as a bile duct, esophagus, duodenum, small intestine, or large intestine, and is used primarily to expand and maintain the lumen. The stent device 100 includes a stent 110 and a cover 120. The stent device 100 is a covered stent in which the stent 110 formed by weaving a wire is covered with a cover 120.

In the following description, one side in a longitudinal axis direction (an axial direction) A of the stent device 100 will be referred to as a "first direction A1," and the other side in the longitudinal axis direction A of the stent device 100 will be referred to as a "second direction A2."

### [Stent 110]

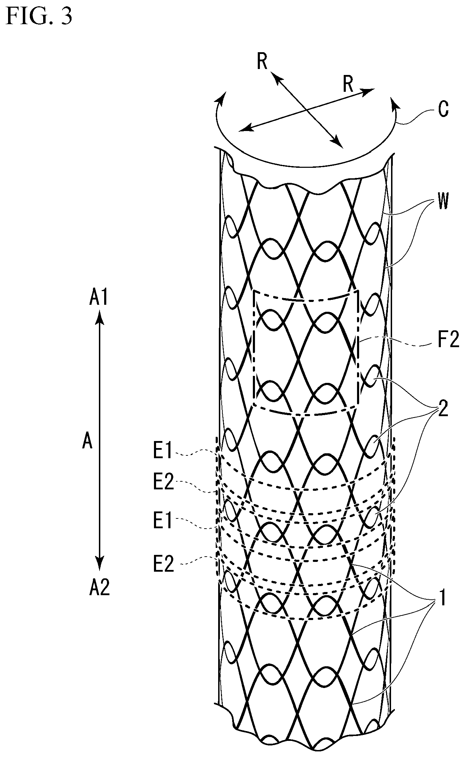

FIG. 3 is a diagram showing the overall configuration of the stent 110.

The stent 110 is formed by weaving a wire and has a cylindrical shape.

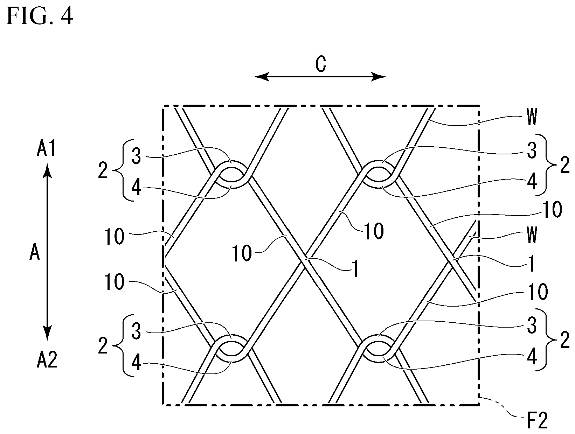

FIG. 4 is a development view of the stent 110 in an F2 region shown in FIG. 3.

The stent 110 is formed into a circular tube shape with a mesh on the circumferential surface by a wire W extending obliquely in a circumferential direction C while repeating bending. The stent 110 has a plurality of straight line intersection portions 1 and a plurality of engagement portions 2.

The straight line intersection portion 1 is formed by straight line portions 10 of the wires W intersecting with each other in a straight line. The straight line portion 10 is a portion of the wire W having a substantially straight line shape, and also includes a gently curved portion.

The engagement portion (an intertwining portion) 2 is formed by a mountain-shaped bent portion 3 and a valley-shaped bent portion 4 intersecting with each other. The mountain-shaped bent portion (a mountain) 3 is a convex portion that is convex toward a side in the first direction A1, in which the wire W extending obliquely in the circumferential direction C is bent back in the longitudinal axis direction A. The valley-shaped bent portion (a valley) 4 is a convex portion that is convex toward a side in the second direction A2 (a concave portion that is concave toward a side in the first direction A1), in which the wire W extending obliquely in the circumferential direction is bent back in the longitudinal axis direction A. At the engagement portion 2, the mountain-shaped bent portion 3 and the valley-shaped bent portion 4 intersect with each other in a hook shape, and thus the mountain-shaped bent portion 3 and the valley-shaped bent portion 4 are connected to each other such that they can be moved relative to each other, although they cannot be separated from each other.

In the present embodiment, the engagement portions 2 adjacent to each other in the circumferential direction C are disposed at substantially the same position in the longitudinal axis direction A. Furthermore, the engagement portions 2 adjacent to each other in the longitudinal axis direction A are disposed at substantially the same position in the circumferential direction C.

As shown in FIG. 3, the plurality of straight line intersection portions 1 are disposed parallel to the circumferential direction C. Further, the plurality of straight line intersection portions 1 are disposed parallel to the circumferential direction C. A first region E1 in which the plurality of straight line intersection portions 1 are disposed and a second region E2 in which the plurality of engagement portions 2 are disposed are alternately disposed in the longitudinal axis direction A.

### [Cover 120]

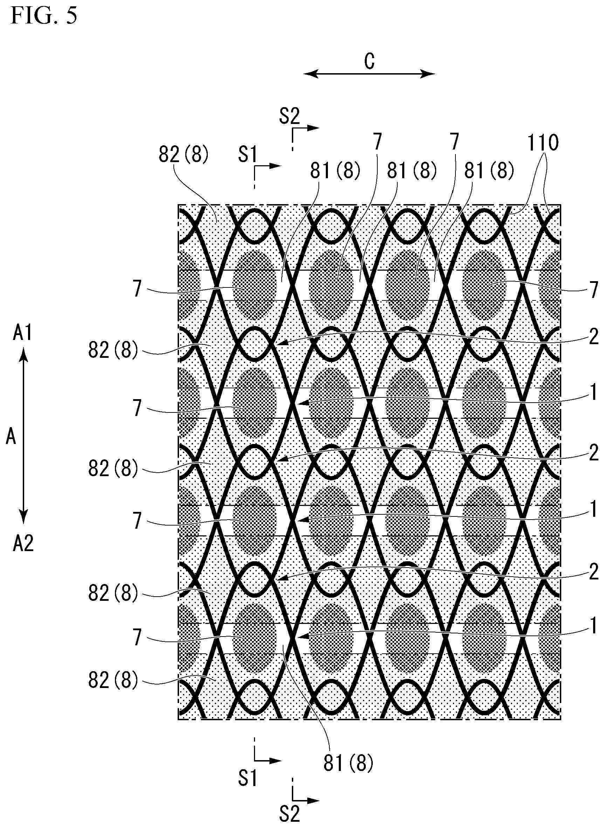

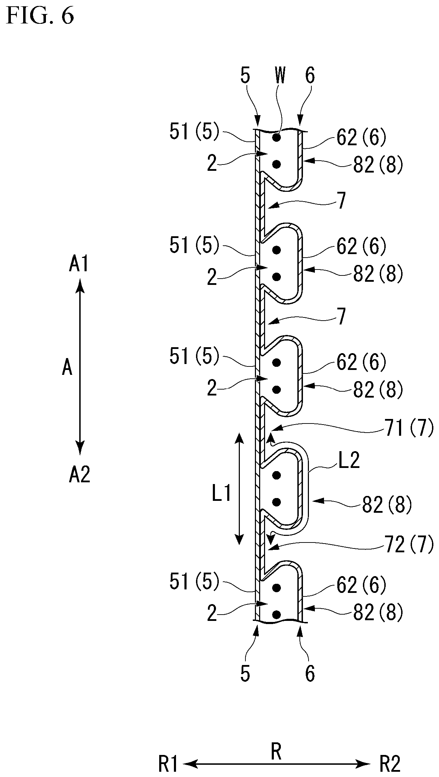

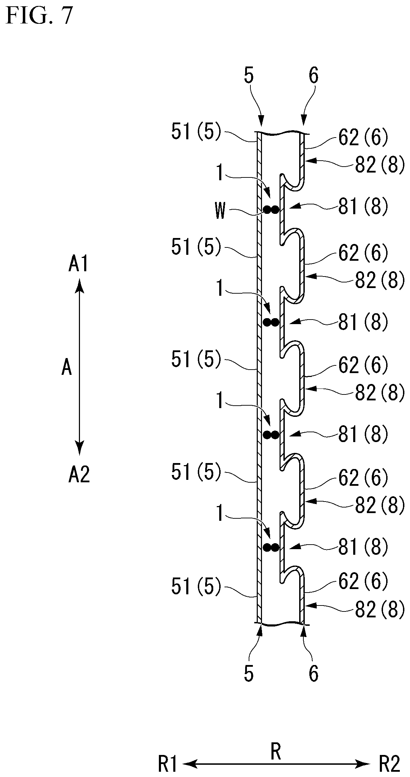

FIG. 5 is a diagram showing the stent device 100 in an F1 region shown in FIG. 2. FIG. 6 is a cross-sectional view along line S1-S1 shown in FIG. 5. FIG. 7 is a cross-sectional view along line S2-S2 shown in FIG. 5. The cover 120 covers the entire stent 110. The cover 120 is made of a flexible material such as polytetrafluoroethylene (PTFE) or polyurethane nonwoven fabric, and can prevent infiltration of cells and blood.

As shown in FIGS. 6 and 7, the cover 120 is formed by overlapping an inside cover 5 disposed on an inside R1 of the stent 110 in a radial direction R and an outside cover 6 disposed on the outside R2 of the stent 110 in the radial direction R each other. The inside cover 5 and the outside cover 6 may be formed integrally or may be formed separately. Specifically, the cover 120 includes a fixation portion 7 at which the inside cover 5 and the outside cover 6 are fixed to each other, and an accommodation portion 8 in which the wire W is accommodated.

As shown in FIG. 5, the fixation portion (a fixation region) 7 is a region sandwiched between the engagement portions 2 adjacent to each other in the longitudinal axis direction A, and is formed into a roughly diamond-shaped region surrounded by the mountain-shaped bent portion 3 and the valley-shaped bent portion 4. As shown in FIG. 6, the fixation portion 7 is a region in which the inside cover 5 and the outside cover 6 are fixed to each other by bonding, pressure bonding, or the like. Since the inside cover 5 and the outside cover 6 are fixed to each other, the wire W cannot pass through the fixation portion 7.

The accommodation portion (a non-fixation region) 8 is a region formed by the inside cover 5 and the outside cover 6 that are not fixed to each other. The accommodation portion 8 accommodates the wire W in an internal region sandwiched between the inside cover 5 and the outside cover 6. As shown in FIGS. 6 and 7, the accommodation portion 8 has a normal accommodation portion 81 and an expanded accommodation portion 82 that is wider than the normal accommodation portion 81 in the radial direction R.

As shown in FIGS. 5 and 7, the normal accommodation portion 81 is formed by the inside cover 5 extending in the longitudinal axis direction A and the outside cover 6 extending in the longitudinal axis direction A. The normal accommodation portion 81 is formed in the first region E1 in which the plurality of straight line intersection portions 1 are disposed in the circumferential direction C. The normal accommodation portion 81 accommodates the straight line intersection portion 1 and the wire W near the straight line intersection portion 1 in an internal region sandwiched between the inside cover 5 and the outside cover 6.

As shown in FIGS. 5 and 6, the expanded accommodation portion 82 is formed by a non-expanded portion 51 that is a portion of the inside cover 5 extending in the longitudinal axis direction A and an externally expanded portion 62 formed by a portion of the outside cover 6 bulging to the outside R2. The expanded accommodation portion 82 is formed in the second region E2 in which the plurality of engagement portions 2 are disposed in the circumferential direction C. The expanded accommodation portion 82 accommodates the plurality of engagement portions 2 disposed in the circumferential direction C in an internal region sandwiched between the inside cover 5 and the outside cover 6. The expanded accommodation portion 82 may be formed separately for each engagement portion 2.

In the cross section along line S1-S1 shown in FIG. 6, the fixation portions 7 and the expanded accommodation portions 82 are alternately disposed one by one. Further, in the cross section along the line S1-S1 shown in FIG. 6, the non-expanded portion 51 of the inside cover 5 closes an opening of the externally expanded portion 62 of the outside cover 6.

As shown in FIG. 6, at least a portion of the externally expanded portion 62 is displaceable to a position at which the at least a portion of the externally expanded portion 62 overlaps the fixation portion 7 when viewed in the radial direction R. In addition, the externally expanded portion 62 of the outside cover 6 may have elasticity, and may be deformed to be displaceable to a position at which the externally expanded portion 62 overlaps the fixation portion 7 when viewed in the radial direction R.

As shown in FIG. 6, the fixation portions 7 disposed on both sides of the expanded accommodation portion 82 in the longitudinal axis direction A are defined as a first fixation portion 71 and a second fixation portion 72. A path length L2 of the outside cover 6 from the first fixation portion 71 to the second fixation portion 72 is longer than a path length L1 of the inside cover 5 from the first fixation portion 71 to the second fixation portion 72.

A surface area of the externally expanded portion 62 of the outside cover 6 forming the expanded accommodation portion 82 is larger than a surface area of the non-expanded portion 51 of the inside cover 5 forming the expanded accommodation portion 82.

In the cross section along line S2-S2 shown in FIG. 7, the normal accommodation portion 81 and the expanded accommodation portions 82 are alternately disposed one by one. In the cross section along line S2-S2 shown in FIG. 7, the internal region of the expanded accommodation portion 82 is connected to the internal region of the normal accommodation portion 81.

### [Operation of stent device 100]

Next, the curving operation of the stent device 100 will be explained. In the following description, the engagement portion 2 disposed on the inside in a curving direction will be referred to as an "inside engagement portion 2A." Moreover, the engagement portion 2 disposed on the outside in the curving direction will be referred to as an "outside engagement portion 2B."

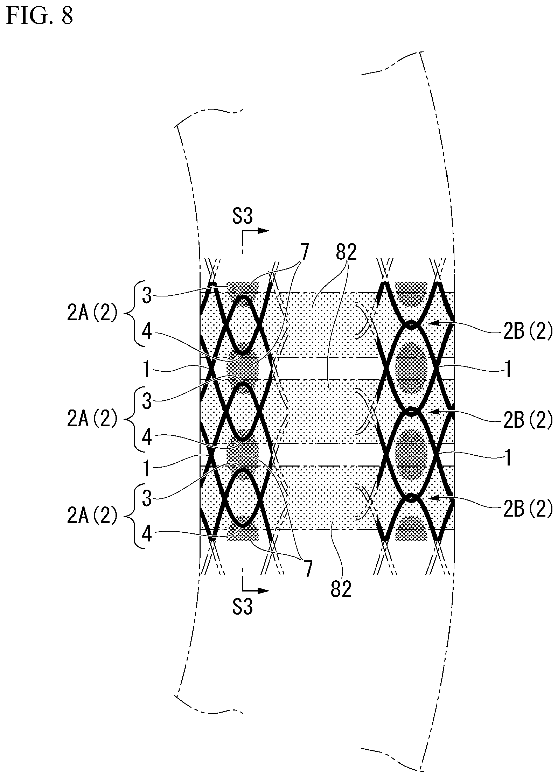

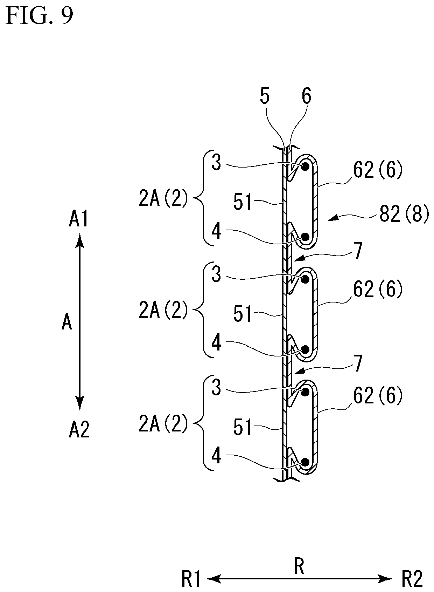

FIG. 8 is a partially transparent view of the stent device 100 curved to one side. FIG. 9 is a cross-sectional view along line S3-S3 shown in FIG. 8. The mountain-shaped bent portion 3 of the inside engagement portion 2A accommodated in the expanded accommodation portion 82 is movable in the first direction A1 to a position at which the mountain-shaped bent portion 3 overlaps the fixation portion 7 when viewed in the radial direction R. The valley-shaped bent portion 4 of the inside engagement portion 2A accommodated in the expanded accommodation portion 82 is movable in the second direction A2 to a position at which the valley-shaped bent portion 4 overlaps the fixation portion 7 when viewed in the radial direction R. For this reason, in the curving operation of the stent device 100, the operation of the mountain-shaped bent portion 3 and the valley-shaped bent portion 4 of the inside engagement portion 2A away from each other is not impeded by the fixation portion 7.

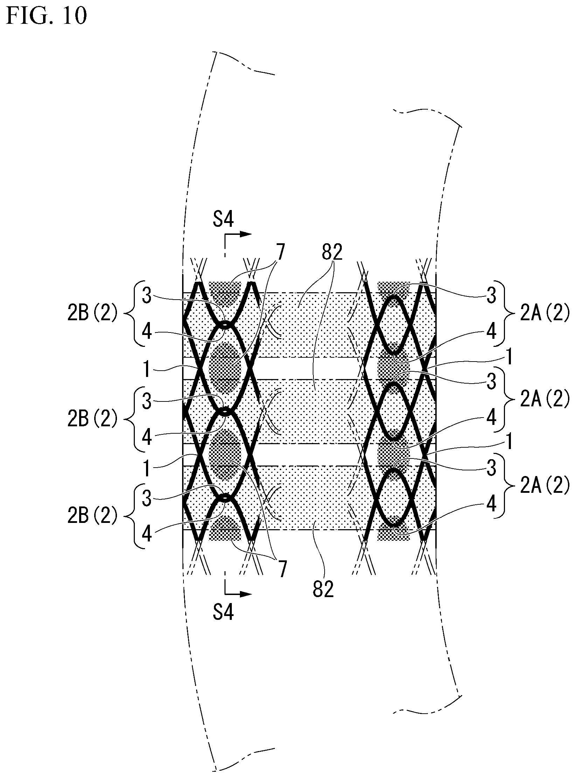

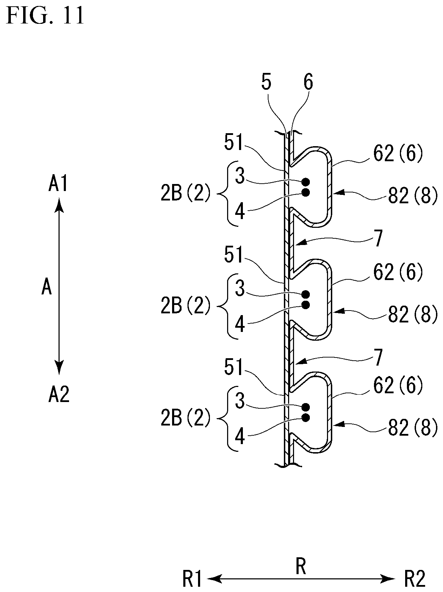

FIG. 10 is a partially transparent view of the stent device 100 curved to the other side. FIG. 11 is a cross-sectional view along line S4-S4 shown in FIG. 10. The mountain-shaped bent portion 3 of the outside engagement portion 2B accommodated in the expanded accommodation portion 82 is movable in the second direction A2 until the mountain-shaped bent portion 3 comes into contact with the valley-shaped bent portion 4. The valley-shaped bent portion 4 of the outside engagement portion 2B accommodated in the expanded accommodation portion 82 is movable in the first direction A1 until the valley-shaped bent portion 4 comes into contact with the mountain-shaped bent portion 3. For this reason, in the curving operation of the stent device 100, the motion of the mountain-shaped bent portion 3 and the valley-shaped bent portion 4 of the outside engagement portion 2B toward each other is not impeded by the fixation portion 7.

### [Operation of stent delivery system 150]

A stent placement method using the endoscope system 300 including the stent delivery system 150 will be described using a technique for placing the stent device 100 in a bile duct as an example.

The operator inserts the insertion section 210 of the endoscope 200 into the patient's body cavity through a natural opening such as the mouth. At that time, the operator operates the knob 223 and the like to curve the curving portion 212 as necessary.

The operator passes a guide wire through the treatment instrument channel 230 of the endoscope 200 and inserts the guide wire into the bile duct while observing with the endoscope 200. Next, the operator operates the guide wire under X-ray fluoroscopy to break through the narrow portion in the bile duct, and moves the distal end portion of the guide wire to the liver side from the narrow portion (a target position).

The operator inserts the proximal end portion of the guide wire protruding from the forceps plug 225 of the endoscope 200 into a through hole of the tip 180 of the stent delivery system 150.

The operator advances the stent delivery system 150 along the guide wire by pushing the stent delivery system 150 while holding the guidewire. The distal end portion of the stent delivery system 150 protrudes from the distal end portion of the treatment instrument channel 230 of the endoscope 200. When the distal end portion of the stent delivery system 150 breaks through the narrow portion (the target position), the operator advances and retracts the stent delivery system 150 to determine the placement position of the stent device 100. The operator may insert the stent delivery system 150 into the treatment instrument channel 230 without using the guide wire.

After the target position of the stent device 100 is determined, the operator retracts the outer tube member 160 with respect to the inner tube member 170. As a result, as shown in FIG. 1, the stent device 100 is gradually exposed and expanded from the distal end side.

When stent device 100 is fully exposed, the stent device 100 is expanded in its entirety such that the inner diameter of the stent device 100 is greater than the outer diameter of inner tube member 170. Accordingly, the engagement between the stent device 100 and the inner tube member 170 is released.

After the engagement between the stent device 100 and the inner tube member 170 is released, when the operator retracts the inner tube member 170, the stent device 100 remains in the placement position and the inner tube member 170 is removed from the stent device 100.

When the operator pulls out the stent delivery system 150 except for the stent device 100 from the body, the procedure for placing the stent device 100 is completed.

According to the stent device 100 according to the present embodiment, even if the stent 110 is covered with the cover 120, the stent device 100 is easily curved. The curving operation is not impeded by the fixation portion 7 whether the engagement portion 2 is disposed on the inside or on the outside in the curving direction.

In the above, the first embodiment of the present invention has been described in detail with reference to the drawings, but the specific configuration is not limited to the embodiment, and a design change and the like within a range not departing from the present invention are also included. In addition, the constituent elements shown in the above-described embodiment and a modification example shown below can be appropriately combined and configured.

### (Modification Example 1-1)

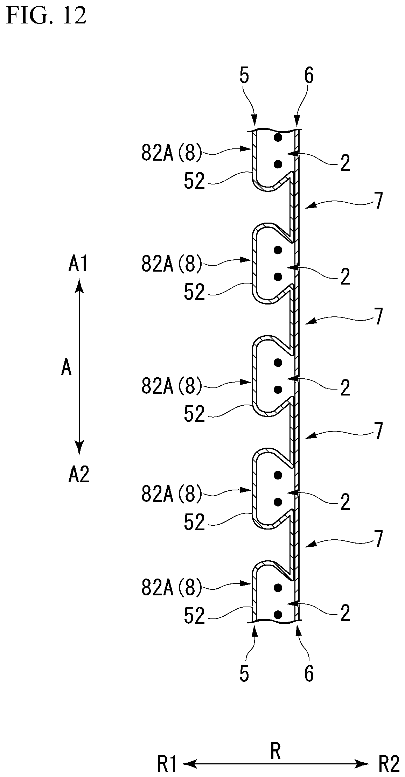

In the present embodiment, the expanded accommodation portion 82 is formed by the non-expanded portion 51 of the inside cover 5 and the externally expanded portion 62 of the outside cover 6. However, the aspect of the expanded accommodation portion 82 is not limited to this. FIG. 12 is a diagram showing an expanded accommodation portion 82A that is a modification example of the expanded accommodation portion 82. The expanded accommodation portion 82A is formed by an internally expanded portion 52 formed by a portion of the inside cover 5 bulging to the inside R1 and a non-expanded portion 61 that is a portion of the outside cover 6 extending in the longitudinal axis direction A.

### (Second embodiment)

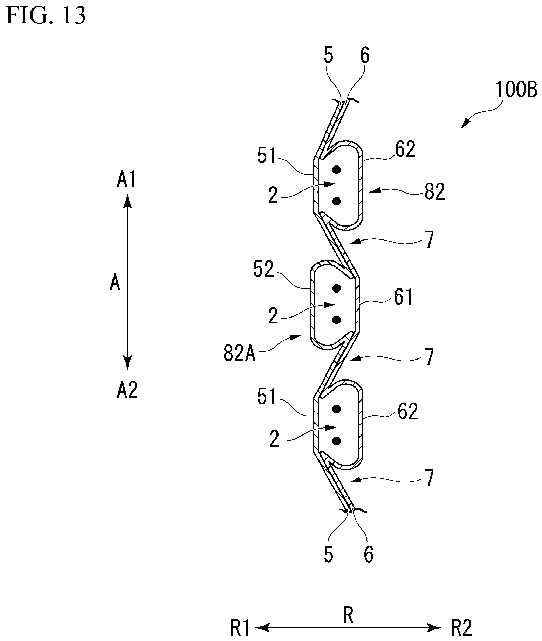

A second embodiment of the present invention will be described with reference to FIGS. 13 to 15. In the following description, the same constituent elements as those already described are designated by the same reference signs, and duplicate description will be omitted. A stent device 100B according to the second embodiment is accommodated in a stent delivery system 150 similarly to the stent device 100 according to the first embodiment. The stent device 100B includes a stent 110 and a cover 120B.

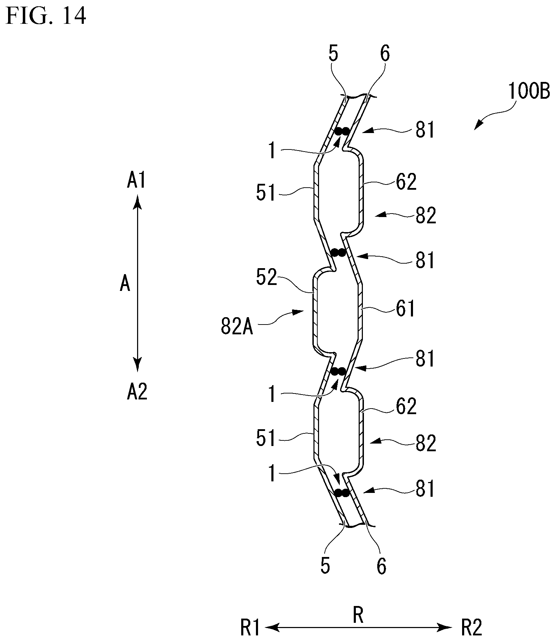

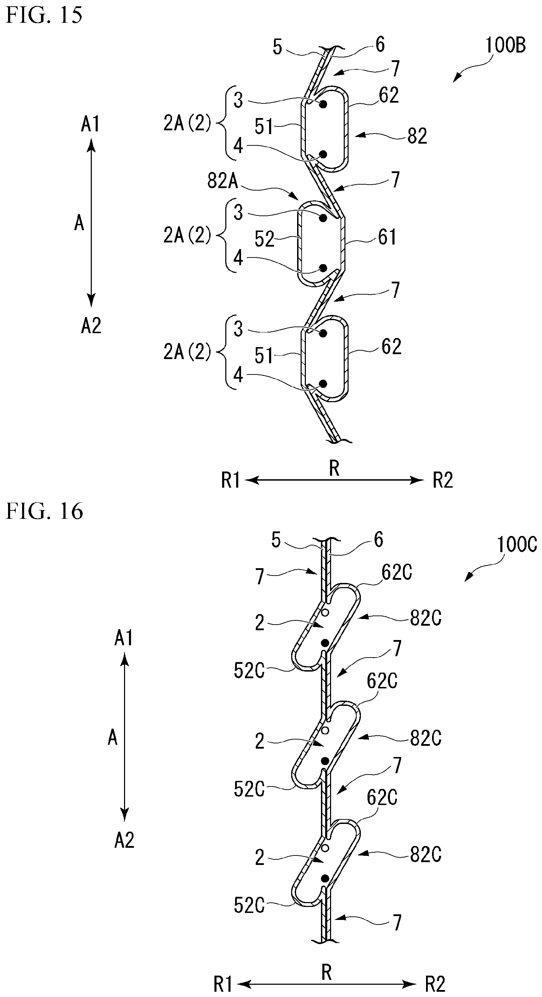

FIG. 13 is a cross-sectional view of the stent device 100B including a fixation portion 7 and expanded accommodation portions 82 and 82A. FIG. 14 is a cross-sectional view of the stent device 100B including a normal accommodation portion 81 and the expanded accommodation portions 82 and 82A. FIG. 15 is a cross-sectional view of the stent device 100B including an inside engagement portion 2A.

As shown in FIGS. 13 and 14, in the cover 120B, the expanded accommodation portions 82 and the expanded accommodation portions 82A are alternately arranged one by one in the longitudinal axis direction A. The cover 120B is difficult to be bulked up in the radial direction R than the cover 120 of the first embodiment. For this reason, the operator easily releases the stent device 100B from the stent delivery system 150. Further, the operator easily recaptures the stent device 100B in the stent delivery system 150.

As shown in FIG. 15, a mountain-shaped bent portion 3 of the inside engagement portion 2A accommodated in the expanded accommodation portion 82 is movable in the first direction A1 to a position at which the mountain-shaped bent portion 3 overlaps the fixation portion 7 when viewed in the radial direction R. A valley-shaped bent portion 4 of the inside engagement portion 2A accommodated in the expanded accommodation portion 82 is movable in the second direction A2 to a position at which the valley shaped bent portion 4 overlaps the fixation portion 7 when viewed in the radial direction R. For this reason, in the curving operation of the stent device 100B, the operation of the mountain-shaped bent portion 3 and the valley-shaped bent portion 4 of the inside engagement portion 2A away from each other is not impeded by the fixation portion 7.

According to the stent device 100B according to the present embodiment, even if the stent 110 is covered with the cover 120B, the stent device 100B is easily curved. The curving operation is not impeded by the fixation portion 7 whether the engagement portion 2 is disposed on the inside or on the outside in the curving direction.

In the above, the second embodiment of the present invention has been described in detail with reference to the drawings, but the specific configuration is not limited to the embodiment, and a design change and the like within a range not departing from the present invention are also included. In addition, the constituent elements shown in the above-described embodiment and a modification example can be appropriately combined and configured.

### (Modification Example 2-1)

In the embodiment described above, the expanded accommodation portions 82 and the expanded accommodation portions 82A are alternately arranged one by one in the longitudinal axis direction A. However, the arrangement aspect of the expanded accommodation portion 82 and the expanded accommodation portion 82A is not limited to this. For example, the expanded accommodation portions 82 and the expanded accommodation portions 82A may be arranged such that two consecutive expanded accommodation portions 82 and one expanded accommodation portion 82A are alternately arranged.

### (Third embodiment)

A third embodiment of the present invention will be described with reference to FIGS. 16 to 18. In the following description, the same constituent elements as those already described are designated by the same reference signs, and duplicate description will be omitted. A stent device 100C according to the third embodiment is accommodated in a stent delivery system 150 similarly to the stent device 100 according to the first embodiment. The stent device 100C includes a stent 110 and a cover 120C.

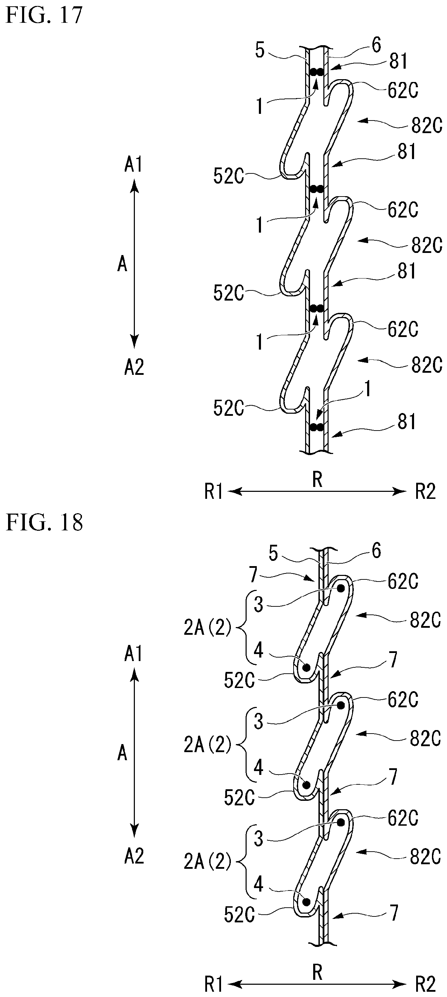

FIG. 16 is a cross-sectional view of the stent device 100C including a fixation portion 7 and an expanded accommodation portion 82C. FIG. 17 is a cross-sectional view of the stent device 100C including a normal accommodation portion 81 and the expanded accommodation portion 82C. FIG. 18 is a cross-sectional view of the stent device 100C including an inside engagement portion 2A.

As shown in FIGS. 16 and 17, the cover 120C has the expanded accommodation portion 82C instead of the expanded accommodation portion 82 of the first embodiment. The expanded accommodation portion 82C is formed by an internally expanded portion 52C formed by a portion of the inside cover 5 bulging to the inside R1 and an externally expanded portion 62C formed by a portion of the outside cover 6 bulging to the outside R2. The expanded accommodation portion 82C accommodates the engagement portion 2 in an internal region sandwiched between the inside cover 5 and the outside cover 6.

The cover 120C is difficult to be bulked up in the radial direction R than the cover 120 of the first embodiment. For this reason, the operator easily releases the stent device 100C from the stent delivery system 150. Further, the operator easily recaptures the stent device 100C in the stent delivery system 150.

As shown in FIG. 18, a mountain-shaped bent portion 3 of the inside engagement portion 2A accommodated in the expanded accommodation portion 82C is movable in the first direction A1 to a position at which the mountain-shaped bent portion 3 overlaps the fixation portion 7 when viewed in the radial direction R. A valley-shaped bent portion 4 of the inside engagement portion 2A accommodated in the expanded accommodation portion 82C is movable in the second direction A2 to a position at which the valley-shaped bent portion 4 overlaps the fixation portion 7 when viewed in the radial direction R. For this reason, in the curving operation of the stent device 100C, the operation of the mountain-shaped bent portion 3 and the valley-shaped bent portion 4 of the inside engagement portion 2A away from each other is not impeded by the fixation portion 7.

According to the stent device 100C according to the present embodiment, even if the stent 110 is covered with the cover 120C, the stent device 100C is easily curved. The curving operation is not impeded by the fixation portion 7 whether the engagement portion 2 is disposed on the inside or on the outside in the curving direction.

In the above, the third embodiment of the present invention has been described in detail with reference to the drawings, but the specific configuration is not limited to the embodiment, and a design change and the like within a range not departing from the present invention are also included. In addition, the constituent elements shown in the above-described embodiment and a modification example can be appropriately combined and configured.

### (Modification Example 3-1)

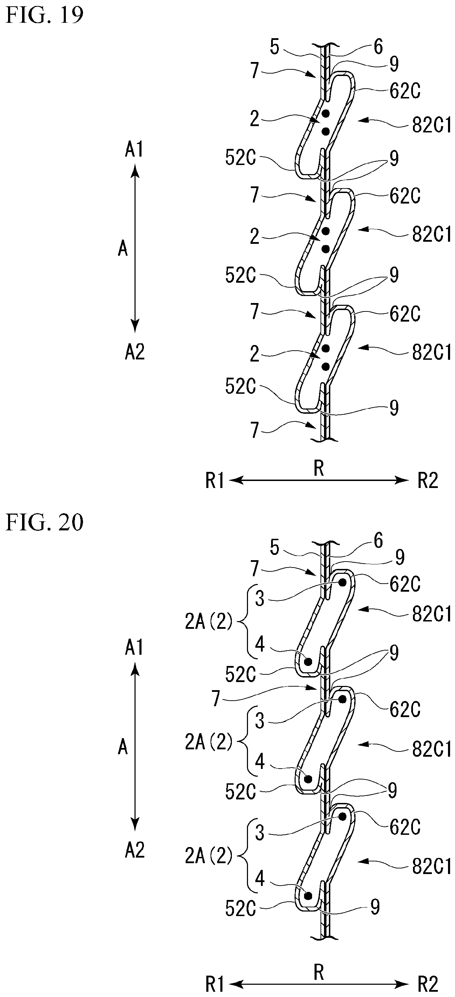

FIGS. 19 and 20 are cross-sectional views of an expanded accommodation portion 82C1 that is a modification example of the expanded accommodation portion 82C. The expanded accommodation portion 82C1 is formed by the internally expanded portion 52C and the externally expanded portion 62C similarly to the expanded accommodation portion 82C. In the expanded accommodation portion 82C1, the externally expanded portion 62C is attached to the fixation portion 7 by a bonding portion 9 while extending in the first direction A1. Further, the internally expanded portion 52C is attached to the fixation portion 7 by the bonding portion 9 while extending in the second direction A2. The expanded accommodation portion 82C1 is difficult to be bulked up in the radial direction R, and does not impede the operation of the engagement portion 2.

### (Modification Example 3-2)

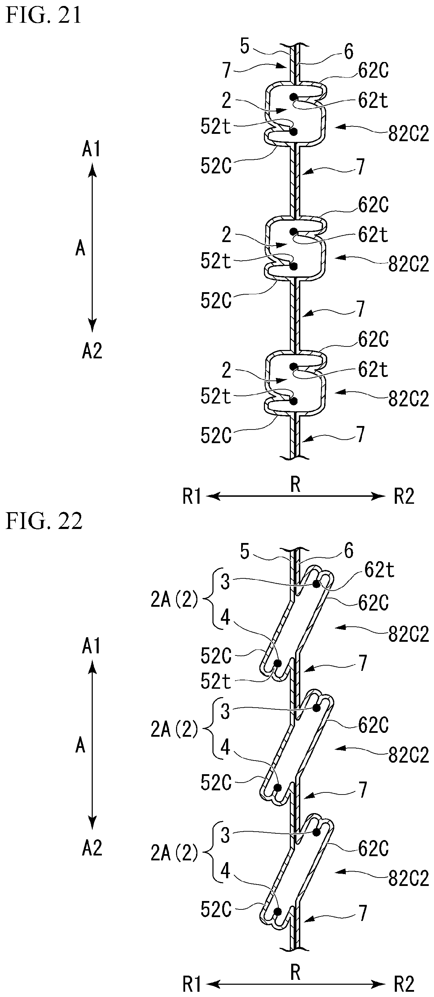

FIGS. 21 and 22 are cross-sectional views of an expanded accommodation portion 82C2 that is a modification example of the expanded accommodation portion 82C. The expanded accommodation portion 82C2 is formed by the internally expanded portion 52C and the externally expanded portion 62C similarly to the expanded accommodation portion 82C. In the expanded accommodation portion 82C2, a distal end portion 62t of the externally expanded portion 62C is fixed to the mountain-shaped bent portion 3. Further, in the expanded accommodation portion 82C2, a distal end portion 52t of the internally expanded portion 52C is fixed to the valley-shaped bent portion 4. As shown in FIG. 22, the expanded accommodation portion 82C1 is difficult to be bulked up in the radial direction R.

### (Fourth embodiment)

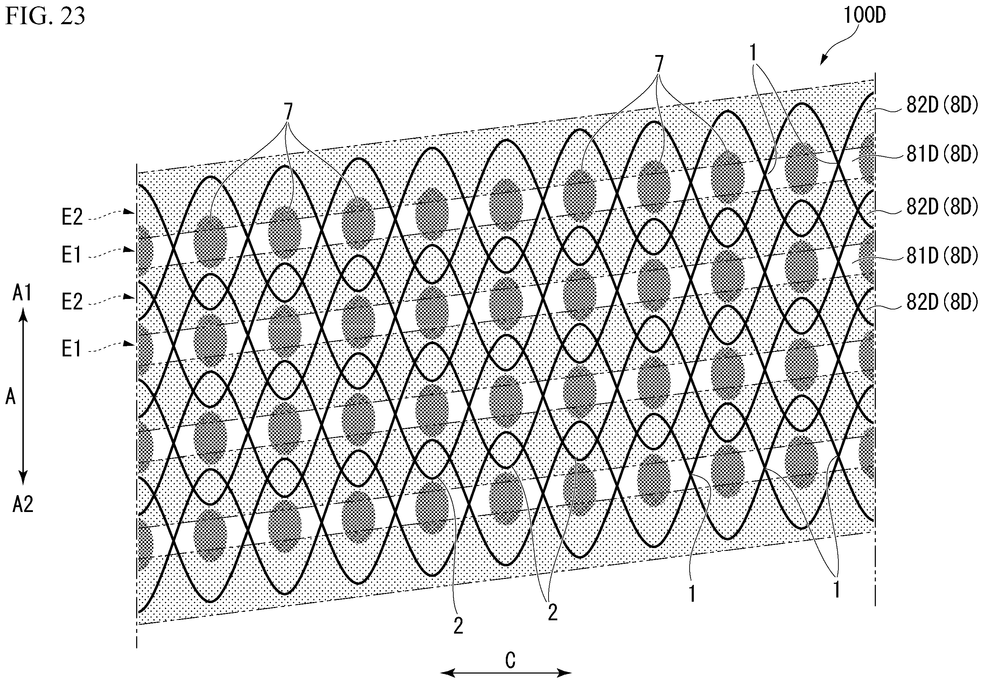

A fourth embodiment of the present invention will be described with reference to FIG. 23. In the following description, the same constituent elements as those already described are designated by the same reference signs, and duplicate description will be omitted. A stent device 100D according to the fourth embodiment is accommodated in a stent delivery system 150 similarly to the stent device 100 according to the first embodiment. The stent device 100D includes a stent 110D and a cover 120D.

FIG. 23 is a development view of the stent device 100D developed in the circumferential direction C.

The stent 110D differs from the stent 110 of the first embodiment only in the arrangement of a plurality of straight line intersection portions 1 and a plurality of engagement portions 2. In the stent 110D, a first region E1 in which the plurality of straight line intersection portions 1 are disposed and a second region E2 in which the plurality of engagement portions 2 are disposed are alternately disposed in the longitudinal axis direction A. The first region E1 is disposed spirally in the longitudinal axis direction A. Further, the second region E2 is disposed spirally in the longitudinal axis direction A.

The cover 120D includes a fixation portion 7 at which an inside cover 5 and an outside cover 6 are fixed to each other, and an accommodation portion 8D in which the wire W is accommodated.

The accommodation portion (a non-fixation region) 8D is a region formed by the inside cover 5 and the outside cover 6 that are not fixed to each other similarly to the accommodation portion 8. The accommodation portion 8D has a normal accommodation portion 81D and an expanded accommodation portion 82D that is wider than the normal accommodation portion 81D in the radial direction R.

The normal accommodation portion 81D is formed in the first region E1 in which the plurality of straight line intersection portions 1 are disposed spirally in the longitudinal axis direction A. The expanded accommodation portion 82D is formed in the second region E2 in which the plurality of engagement portions 2 are disposed spirally in the longitudinal axis direction A.

According to the stent device 100D according to the present embodiment, even if the stent 110D is covered with the cover 120D, the stent device 100D is easily curved. The curving operation is not impeded by the fixation portion 7 whether the engagement portion 2 is disposed on the inside or on the outside in the curving direction.

In the above, the fourth embodiment of the present invention has been described in detail with reference to the drawings, but the specific configuration is not limited to the embodiment, and a design change and the like within a range not departing from the present invention are also included. In addition, the constituent elements shown in the above-described embodiment and a modification example can be appropriately combined and configured.

### (Modification Example 4-1)

A method of weaving the stent is not limited to that of the stent 110 of the first embodiment or that of the stent 110D of the fourth embodiment. Other methods of weaving the stent may be used.

### (Fifth embodiment)

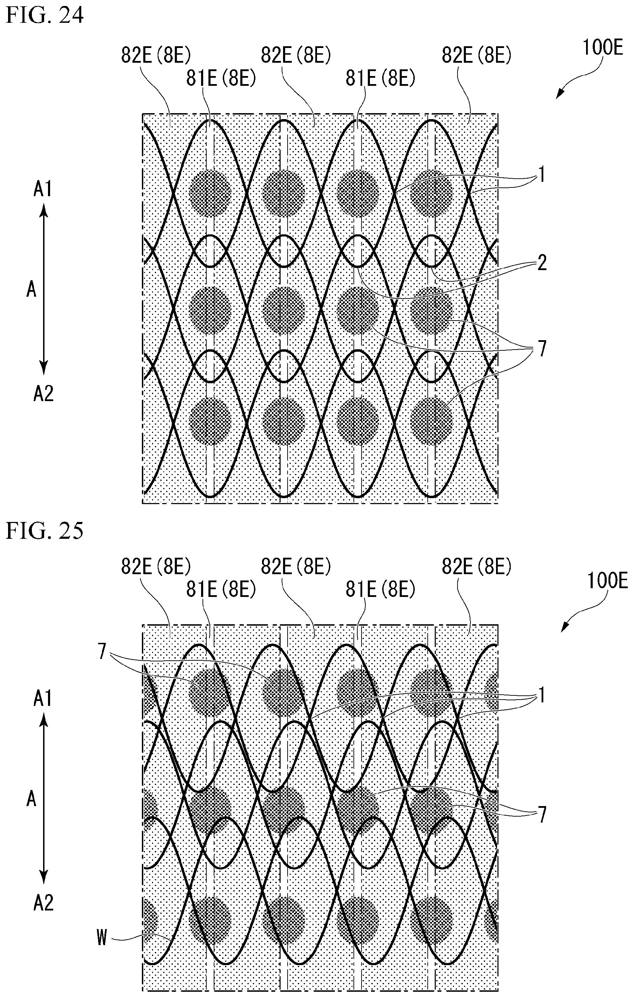

A fifth embodiment of the present invention will be described with reference to FIGS. 24 and 25. In the following description, the same constituent elements as those already described are designated by the same reference signs, and duplicate description will be omitted. A stent device 100E according to the fifth embodiment is accommodated in a stent delivery system 150 similarly to the stent device 100 according to the first embodiment. The stent device 100E includes a stent 110 and a cover 120E.

FIG. 24 is a diagram showing a portion of the stent device 100E.

The cover 120E is formed by overlapping an inside cover 5 disposed on an inside R1 of the stent 110 in a radial direction R and an outside cover 6 disposed on the outside R2 of the stent 110 in the radial direction R each other similarly to the cover 120 of the first embodiment. Specifically, the cover 120E includes a fixation portion 7 at which the inside cover 5 and the outside cover 6 are fixed to each other, and an accommodation portion 8E in which the wire W is accommodated.

The accommodation portion (a non-fixation region) 8E is a region formed by the inside cover 5 and the outside cover 6 that are not fixed to each other similarly to the accommodation portion 8. The accommodation portion 8E has a normal accommodation portion 81E and an expanded accommodation portion 82E that is wider than the normal accommodation portion 81E in the radial direction R.

The normal accommodation portion 81E is formed in a third region E3 in which the plurality of engagement portions 2 are arranged in the longitudinal axis direction A. The expanded accommodation portion 82E is formed in a fourth region E4 in which the plurality of straight line intersection portions 1 are arranged in the longitudinal axis direction A. The expanded accommodation portion 82E accommodates the plurality of straight line intersection portions 1 disposed in the longitudinal axis direction A and the wires W near the straight line intersection portions 1 in an internal region sandwiched between the inside cover 5 and the outside cover 6.

FIG. 25 is a diagram showing a portion of the stent device 100E twisted.

Even in a case where the stent device 100E is twisted, as shown in FIG. 25, the straight line intersection portion 1 and the wire W near the straight line intersection portion 1 are movable to a position at which they overlap the fixation portion 7 when viewed in the radial direction R.

According to the stent device 100E according to the present embodiment, even if the stent 110 is covered with the cover 120D, the stent device 100E is easily curved.

In the above, the fifth embodiment of the present invention has been described in detail with reference to the drawings, but the specific configuration is not limited to the embodiment, and a design change and the like within a range not departing from the present invention are also included. In addition, the constituent elements shown in the above-described embodiment and a modification example can be appropriately combined and configured.

### (Modification Example 5-1)

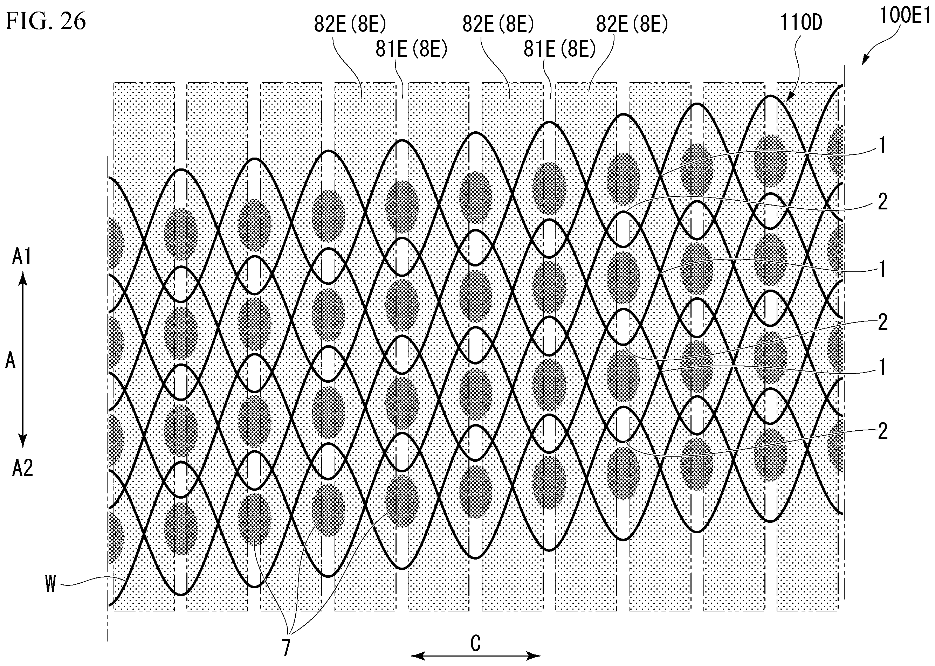

FIG. 26 is a development view of a stent device 100E1 that is a modification example of the stent device 100E. The stent device 100E1 includes a stent 110D and a cover 120E. The expanded accommodation portion 82E is formed as a continuous region in the longitudinal axis direction A. For this reason, even in a case where the straight line intersection portion 1 and the engagement portion 2 are disposed in a spiral in the longitudinal axis direction A as in the stent 110D, the manufacturing cost of the expanded accommodation portion 82E is low.

### [Industrial Applicability]

The present invention can be applied to a stent covered with a cover.

### [Reference Signs List]

300 Endoscope system
200 Endoscope
150 Stent delivery system
140 Operation part
160 Outer tube member
170 Inner tube member
180 Tip
100, 100B, 100C, 100D, 100E Stent device
110, 110D Stent
120, 120B, 120C, 120D, 120E Cover
1 Straight line intersection portion
10 Straight line portion
2 Engagement portion (intertwining portion)
3 Mountain-shaped bent portion (mountain)
4 Valley-shaped bent portion (valley)
5 Inside cover
6 Outside cover
7 Fixation portion
8 Accommodation portion
81 Normal accommodation portion
82 Expanded accommodation portion

## Claims

1. A stent device (100) comprising: a stent (110) formed in a cylindrical shape by weaving a wire (W); and a cover (120) formed by overlapping an inside cover (5) disposed on an inside of the stent and an outside cover (6) disposed on an outside of the stent (110), wherein the cover (120) has a fixation portion (7) at which the inside cover (5) and the outside cover (6) are fixed to each other, and an accommodation portion (8) which is formed by the inside cover (5) and the outside cover (6) and accommodates the wire (W), and wherein the accommodation portion (8) has a normal accommodation portion and an expanded accommodation portion that is wider than the normal accommodation portion in a radial direction, wherein the stent (110) has an engagement portion (2) at which a mountain-shaped bent portion (3) that is bent toward a side in a first direction, which is one side in an axial direction, and becomes convex, and a valley-shaped bent portion (4) that is bent toward a side in a second direction, which is the other side in the axial direction, and becomes convex intersect with each other, and wherein the expanded accommodation portion accommodates the engagement portion (2).

2. The stent device according to claim 1, wherein the expanded accommodation portion is displaceable to a position at which at least a portion thereof overlaps the fixation portion when viewed in the radial direction.

3. The stent device according to claim 1,
wherein the outside cover has an externally expanded portion formed by a portion of the outside cover bulging to the outside, and
wherein the expanded accommodation portion is formed by the externally expanded portion of the outside cover and a portion of the inside cover extending in an axial direction.

4. The stent device according to claim 3, wherein at least a portion of the externally expanded portion is displaceable to a position at which the at least a portion of the externally expanded portion overlaps the fixation portion when viewed in the radial direction.

5. The stent device according to claim 3,
wherein the expanded accommodation portion is disposed between a first fixation portion and a second fixation portion, each of which is the fixation portion, and
wherein a path length of the outside cover from the first fixation portion to the second fixation portion is longer than a path length of the inside cover from the first fixation portion to the second fixation portion.

6. The stent device according to claim 3, wherein a surface area of the externally expanded portion of the outside cover forming the expanded accommodation portion is larger than a surface area of the portion of the inside cover forming the expanded accommodation portion.

7. The stent device according to claim 1,
wherein the outside cover has an externally expanded portion formed by a portion of the outside cover bulging to the outside,
wherein the inside cover has an internally expanded portion formed by a portion of the inside cover bulging to the inside, and
wherein the expanded accommodation portion is formed by the externally expanded portion of the outside cover and the internally expanded portion of the inside cover.

8. The stent device according to claim 1, wherein the fixation portion and the expanded accommodation portion are arranged in the axial direction.

9. The stent device according to claim 8, wherein the mountain-shaped bent portion accommodated in the expanded accommodation portion is movable in the first direction to a position at which the mountain-shaped bent portion overlaps the fixation portion when viewed in the radial direction.

10. The stent device according to claim 8, wherein the valley-shaped bent portion accommodated in the expanded accommodation portion is movable in the second direction to a position at which the valley-shaped bent portion overlaps the fixation portion when viewed in the radial direction.

11. The stent device according to any one of claims 1 to 10,
wherein a plurality of the engagement portions are arranged in a circumferential direction, and
wherein the expanded accommodation portion is formed in a region continuous in the circumferential direction and accommodates the plurality of engagement portions.

12. The stent device according to claim 1,
wherein the stent has a straight line intersection portion at which straight line portions intersect with each other, and
wherein the normal accommodation portion accommodates the straight line intersection portion.

13. A stent delivery system (150) comprising: an operation part (140); an outer tube member (160) configured to extend to a distal side from the operation part (140); an inner tube member (170) configured to extend to a distal side from the
operation part (140) and located on an inside of the outer tube member (160); and the stent device (100) according to any one of claims 1 to 12 which is accommodated between the outer tube member (160) and the inner tube member (170), wherein the operation part (140) is configured to place the stent device (100) by moving the outer tube member (160) or the inner tube member (170) in a longitudinal direction.

## Patentansprüche

1. Eine Stentvorrichtung (100), umfassend: einen Stent (110), der durch Flechten eines Drahts (W) in einer zylindrischen Form gebildet ist; und eine Abdeckung (120), die durch Überlappen einer inneren Abdeckung (5), die an einer Innenseite des Stents angeordnet ist, und einer äußeren Abdeckung (6), die an einer Außenseite des Stents (110) angeordnet ist, gebildet ist, wobei die Abdeckung (120) einen Befestigungsabschnitt (7) hat, an dem die innere Abdeckung (5) und die äußere Abdeckung (6) aneinander befestigt sind, und einen Aufnahmeabschnitt (8), der durch die innere Abdeckung (5) und die äußere Abdeckung (6) gebildet ist und den Draht (W) aufnimmt, und wobei der Aufnahmeabschnitt (8) einen normalen Aufnahmeabschnitt und einen erweiterten Aufnahmeabschnitt hat, der in einer Radialrichtung breiter ist als der normale Aufnahmeabschnitt, wobei der Stent (110) einen Eingriffsabschnitt (2) hat, an dem sich ein bergförmiger gebogener Abschnitt (3), der zu einer Seite in einer ersten Richtung gebogen ist, die eine Seite in einer Axialrichtung ist, und konvex wird, und ein talförmiger gebogener Abschnitt (4), der zu einer Seite in einer zweiten Richtung gebogen ist, die die andere Seite in der Axialrichtung ist, und konvex wird, miteinander schneiden, und wobei der erweiterte Aufnahmeabschnitt den Eingriffsabschnitt (2) aufnimmt.

2. Die Stentvorrichtung nach Anspruch 1, wobei der erweiterte Aufnahmeabschnitt zu einer Position verschiebbar ist, an der sich zumindest ein Teil davon, in der Radialrichtung betrachtet, mit dem Befestigungsabschnitt überlappt.

3. Die Stentvorrichtung nach Anspruch 1,
wobei die äußere Abdeckung einen nach außen erweiterten Abschnitt hat, der dadurch gebildet ist, dass ein Teil der äußeren Abdeckung nach außen ausbaucht, und
wobei der erweiterte Aufnahmeabschnitt durch den nach außen erweiterten Abschnitt der äußeren Abdeckung und einen sich in einer Axialrichtung erstreckenden Teil der inneren Abdeckung gebildet ist.

4. Die Stentvorrichtung nach Anspruch 3, wobei zumindest ein Teil des nach außen erweiterten Abschnitts zu einer Position verschiebbar ist, an der sich der zumindest eine Teil des nach außen erweiterten Abschnitts, in der Radialrichtung betrachtet, mit dem Befestigungsabschnitt überlappt.

5. Die Stentvorrichtung nach Anspruch 3,
wobei der erweiterte Aufnahmeabschnitt zwischen einem ersten Befestigungsabschnitt und einem zweiten Befestigungsabschnitt angeordnet ist, von denen jeder der Befestigungsabschnitt ist, und
wobei eine Pfadlänge der äußeren Abdeckung von dem ersten Befestigungsabschnitt zu dem zweiten Befestigungsabschnitt länger ist als eine Pfadlänge der inneren Abdeckung von dem ersten Befestigungsabschnitt zu dem zweiten Befestigungsabschnitt.

6. Die Stentvorrichtung nach Anspruch 3, wobei eine Oberflächenfläche des nach außen erweiterten Abschnitts der äußeren Abdeckung, der den erweiterten Aufnahmeabschnitt bildet, größer ist als eine Oberflächenfläche des Teils der inneren Abdeckung, der den erweiterten Aufnahmeabschnitt bildet.

7. Die Stentvorrichtung nach Anspruch 1,
wobei die äußere Abdeckung einen nach außen erweiterten Abschnitt hat, der dadurch gebildet ist, dass ein Teil der äußeren Abdeckung nach außen ausbaucht,
wobei die innere Abdeckung einen nach innen erweiterten Abschnitt hat, der dadurch gebildet ist, dass ein Teil der inneren Abdeckung nach innen ausbaucht, und
wobei der erweiterte Aufnahmeabschnitt durch den nach außen erweiterten Abschnitt der äußeren Abdeckung und den nach innen erweiterten Abschnitt der inneren Abdeckung gebildet ist.

8. Die Stentvorrichtung nach Anspruch 1, wobei der Befestigungsabschnitt und der erweiterte Aufnahmeabschnitt in der Axialrichtung angeordnet sind.

9. Die Stentvorrichtung nach Anspruch 8, wobei der in dem erweiterten Aufnahmeabschnitt aufgenommene bergförmige gebogene Abschnitt in der ersten Richtung zu einer Position beweglich ist, an der sich der bergförmige gebogene Abschnitt, in der Radialrichtung betrachtet, mit dem Befestigungsabschnitt überlappt.

10. Die Stentvorrichtung nach Anspruch 8, wobei der in dem erweiterten Aufnahmeabschnitt aufgenommene talförmige gebogene Abschnitt in der zweiten Richtung zu einer Position beweglich ist, an der sich der talförmige gebogene Abschnitt, in der Radialrichtung betrachtet, mit dem Befestigungsabschnitt überlappt.

11. Die Stentvorrichtung nach einem der Ansprüche 1 bis 10,
wobei eine Mehrzahl der Eingriffsabschnitte in einer Umfangsrichtung angeordnet ist, und
wobei der erweiterte Aufnahmeabschnitt in einem in der Umfangsrichtung zusammenhängenden Bereich gebildet ist und die Mehrzahl der Eingriffsabschnitte aufnimmt.

12. Die Stentvorrichtung nach Anspruch 1,
wobei der Stent einen Geradenschnittabschnitt hat, an dem gerade Linienabschnitte einander schneiden, und
wobei der normale Aufnahmeabschnitt den Geradenschnittabschnitt aufnimmt.

13. Ein Stentabgabesystem (150), umfassend: einen Betätigungsteil (140); ein äußeres Rohrglied (160), das dazu ausgelegt ist, sich von dem Betätigungsteil (140) zu einer distalen Seite zu erstrecken; ein inneres Rohrglied (170), das dazu ausgelegt ist, sich von dem Betätigungsteil (140) zu einer distalen Seite zu erstrecken und an einer Innenseite des äußeren Rohrglieds (160) angeordnet ist; und die Stentvorrichtung (100) nach einem der Ansprüche 1 bis 12, die zwischen dem äußeren Rohrglied (160) und dem inneren Rohrglied (170) aufgenommen ist, wobei der Betätigungsteil (140) dazu ausgelegt ist, die Stentvorrichtung (100) zu platzieren, indem das äußere Rohrglied (160) oder das innere Rohrglied (170) in einer Längsrichtung bewegt wird.

## Revendications

1. Un dispositif de stent (100) comprenant : un stent (110) formé en une forme cylindrique par tissage d'un fil (W) ; et un revêtement (120) formé par superposition d'un revêtement interne (5) disposé à l'intérieur du stent et d'un revêtement externe (6) disposé à l'extérieur du stent (110), dans lequel le revêtement (120) comporte une portion de fixation (7) au niveau de laquelle le revêtement interne (5) et le revêtement externe (6) sont fixés l'un à l'autre, et une portion de logement (8) qui est formée par le revêtement interne (5) et le revêtement externe (6) et loge le fil (W), et dans lequel la portion de logement (8) comporte une portion de logement normale et une portion de logement élargie qui est plus large que la portion de logement normale dans une direction radiale, dans lequel le stent (110) comporte une portion d'engagement (2) au niveau de laquelle une portion courbée en forme de montagne (3) qui est courbée vers un côté dans une première direction, qui est un côté dans une direction axiale, et devient convexe, et une portion courbée en forme de vallée (4) qui est courbée vers un côté dans une seconde direction, qui est l'autre côté dans la direction axiale, et devient convexe, s'entrecroisent, et dans lequel la portion de logement élargie loge la portion d'engagement (2).

2. Le dispositif de stent selon la revendication 1, dans lequel la portion de logement élargie est déplaçable vers une position à laquelle au moins une partie de celle-ci chevauche la portion de fixation lorsqu'elle est vue dans la direction radiale.

3. Le dispositif de stent selon la revendication 1,
dans lequel le revêtement externe comporte une portion élargie vers l'extérieur formée par une partie du revêtement externe faisant saillie vers l'extérieur, et
dans lequel la portion de logement élargie est formée par la portion élargie vers l'extérieur du revêtement externe et une partie du revêtement interne s'étendant dans une direction axiale.

4. Le dispositif de stent selon la revendication 3, dans lequel au moins une partie de la portion élargie vers l'extérieur est déplaçable vers une position à laquelle ladite au moins une partie de la portion élargie vers l'extérieur chevauche la portion de fixation lorsqu'elle est vue dans la direction radiale.

5. Le dispositif de stent selon la revendication 3,
dans lequel la portion de logement élargie est disposée entre une première portion de fixation et une seconde portion de fixation, chacune constituant la portion de fixation, et
dans lequel une longueur de trajet du revêtement externe depuis la première portion de fixation jusqu'à la seconde portion de fixation est plus grande qu'une longueur de trajet du revêtement interne depuis la première portion de fixation jusqu'à la seconde portion de fixation.

6. Le dispositif de stent selon la revendication 3, dans lequel la surface de la portion élargie vers l'extérieur du revêtement externe formant la portion de logement élargie est supérieure à la surface de la partie du revêtement interne formant la portion de logement élargie.

7. Le dispositif de stent selon la revendication 1,
dans lequel le revêtement externe comporte une portion élargie vers l'extérieur formée par une partie du revêtement externe faisant saillie vers l'extérieur,
dans lequel le revêtement interne comporte une portion élargie vers l'intérieur formée par une partie du revêtement interne faisant saillie vers l'intérieur, et
dans lequel la portion de logement élargie est formée par la portion élargie vers l'extérieur du revêtement externe et la portion élargie vers l'intérieur du revêtement interne.

8. Le dispositif de stent selon la revendication 1, dans lequel la portion de fixation et la portion de logement élargie sont disposées dans la direction axiale.

9. Le dispositif de stent selon la revendication 8, dans lequel la portion courbée en forme de montagne logée dans la portion de logement élargie est mobile dans la première direction jusqu'à une position à laquelle la portion courbée en forme de montagne chevauche la portion de fixation lorsqu'elle est vue dans la direction radiale.

10. Le dispositif de stent selon la revendication 8, dans lequel la portion courbée en forme de vallée logée dans la portion de logement élargie est mobile dans la seconde direction jusqu'à une position à laquelle la portion courbée en forme de vallée chevauche la portion de fixation lorsqu'elle est vue dans la direction radiale.

11. Le dispositif de stent selon l'une quelconque des revendications 1 à 10,
dans lequel une pluralité de portions d'engagement sont disposées dans une
direction circonférentielle, et
dans lequel la portion de logement élargie est formée dans une région continue dans la direction circonférentielle et loge la pluralité de portions d'engagement.

12. Le dispositif de stent selon la revendication 1,
dans lequel le stent comporte une portion d'intersection de lignes droites au niveau de laquelle des portions de ligne droite s'intersectent, et
dans lequel la portion de logement normale loge la portion d'intersection de lignes droites.

13. Un système de pose de stent (150) comprenant : une partie d'actionnement (140) ; un élément tubulaire externe (160) configuré pour s'étendre vers un côté distal à partir de la partie d'actionnement (140) ; un élément tubulaire interne (170) configuré pour s'étendre vers un côté distal à partir de la partie d'actionnement (140) et situé à l'intérieur de l'élément tubulaire externe (160) ; et le dispositif de stent (100) selon l'une quelconque des revendications 1 à 12 qui est logé entre l'élément tubulaire externe (160) et l'élément tubulaire interne (170), dans lequel la partie d'actionnement (140) est configurée pour mettre en place le dispositif de stent (100) en déplaçant l'élément tubulaire externe (160) ou l'élément tubulaire interne (170) dans une direction longitudinale.
